# EUROPÄISCHE PATENTSCHRIFT

(11) **EP 0 355 261 B1**
(45) Veröffentlichungstag und Bekanntmachung des Hinweises auf die Patenterteilung: **24.11.1993**
(21) Anmeldenummer: 89107251.4
(22) Anmeldetag: 21.04.1989
(51) Int. Cl.: A61N 2/12

(54) **Einrichtung zur Behandlung von Erkrankungen, insbesondere der Sehbahn**
Device for the treatment of diseases, especially of the ocular system
Dispositif de traitement des maladies, en particulier du système oculaire

(30) Priorität: 18.08.1988 SU 4469065
(43) Veröffentlichungstag der Anmeldung: 28.02.1990
(73) Patentinhaber: MEZHOTRASLEVOI NAUCHNOTEKHNICHESKY KOMPLEX "MIKROKHIRURGIA GLAZA", Moskau (SU)
(72) Erfinder: Fedorov, Svyatoslav Nikolaevich, Dr., Moscow (SU); Linnik, Leonid Feodosievich, Dr., Moscow (SU); Antropov, Gennady Mikhailovich, Moscow (SU); Arnautov, Leonid Nikolaevich, Dr., Moscow (SU); Ippolitov, Vladimir Vasilievich, Moscow (SU); Streltsov, Valentin Fedorovich, Moscow (SU); Stromakov, Alexandr Petrovich, Moscow (SU); Shigina, Nina Alexeevna, Dr., Moscow (SU)
(74) Vertreter: Zellentin, Rüdiger

(56) Entgegenhaltungen:
- FR-A- 1 073 155
- SU-A- 1 139 446

## Beschreibung

Die Erfindung betrifft eine Einrichtung zur Behandlung von Erkrankungen, insbesondere der Sehbahn, mit einem Gehäuse, in dem ein Antrieb und mindestens ein Hauptdauermagnet untergebracht sind, dessen Achse mit einer Welle des Antriebs verbunden ist.

Aus der SU-S 11 39 446 ist bereits ein Applikator zur Magnetfeldtherapie des Auges bekannt, der ein Gehäuse und ein Magnetelement aufweist. Der Applikator ist mit einem Koordinateneinstellring und einer Reihe zusätzlicher Magnetelemente versehen. Die Magnetelemente haben Endstücke und sind in Nuten des Gehäuses so angeordnet, daß sie in Radial- und Längsrichtung verstellbar sind und sich drehen können. Das Gehäuse ist im Koordinateneinstellring drehbar angeordnet.

Der Applikator kann nur frontal am vorderen Abschnitt des Auges, nicht jedoch in anderen Bereichen angeordnet werden, wo eine magnetische Einwirkung nötig ist, so daß er zur Behandlung von Erkrankungen der ganzen Sehbahn nicht einsetzbar ist. Außerdem läßt der Applikator eine synchrone Beeinflussung des Magnetfeldes mit den Pulsationen des Blutstroms in der inneren Kopfschlagader nicht zu, was den Heilungsprozeß verzögert.

Bekannt ist ferner eine magnetische therapeutische Einrichtung (DE-A 32 21 544), die ein Gehäuse aufweist, in dem ein Dauermagnet und ein Antrieb angeordnet sind, dessen Welle mit der Achse des Dauermagneten verbunden ist.

Bei einer Drehung des Dauermagneten wird ein magnetisches Drehfeld um ihn herum erzeugt, das ein Einwirken auf verschiedene pathologische Bereiche ermöglicht. Die Drehung des Dauermagneten erfolgt gleichmäßig, wodurch ein sich sinusförmig änderndes Magnetfeld erzeugt wird, was die Behandlungsdauer verlängert und die Behandlungseffektivität herabsetzt. Bei der Synchronisierung der Drehung des Dauermagneten und der Pulsationen des Blutstromes in der inneren Kopfschlagader entstehen bei dieser Einrichtung keine wesentlichen elektrischen Induktionsfelder, denn für die Entstehung beträchtlicher elektrischer Felder sind sehr große Induktionen des Magnetfeldes (in der Größenordnung 1 T) erforderlich, was mit der bekannten Einrichtung nicht auszuführen ist. Der Einsatz der bekannren Einrichtung verursacht Nebenerscheinungen, wie Kopfschmerzen und gesteigerten Augeninnendruck, was die Behandlungseffektivität der Sehbahn vermindert.

Der Erfindung liegt die Aufgabe zugrunde, die Einrichtung zur Behandlung von Erkrankungen der Sehbahn der gattungsgemäßen Art so auszubilden, daß zur Verbesserung der Effektivität der Behandlung eine Änderung des magnetischen Drehfeldes bezüglich Amplitude und Richtung durchführbar ist.

Diese Aufgabe wird erfindungsgemäß durch eine Magnetbremse gelöst, die im Gehäuse in der Wirkungszone des Magnetfeldes untergebracht ist, wobei die Achse des Dauermagneten mit der Welle des Antriebs durch ein elastisches Element verbunden ist.

Zweckmäßigerweise ist die Magnetbremse in der Form von mindestens einem magnetischen Stahlstäbchen ausgeführt. Sie kann jedoch auch in der Form von mindestens einem Dauermagnet ausgeführt werden.

Vorteilhafterweise wird ein Schirm vorgesehen, der ein Fenster hat und der um den Hauptdauermagnet herum angeordnet und aus einem Stoff von hoher elektrischer Leitfähigkeit hergestellt ist.

Die erfindungsgemäße Einrichtung zur Behandlung von Erkrankungen der Sehbahn ermöglicht es, die Sehfunktion entsprechend der Ätiologie der Krankheit auf das zwei-bis fünfzehnfache der Sehschärfe nach zu vergrößern, die Behandlungseffektivität zu verbessern und die Behandlungsdauer auf 15 Tage - 1 Zyklus (15 Prozeduren der Magnetotherapie) zu reduzieren.

Die erfindungsgemäße Einrichtung kann auch bei der Heilung anderer Krankheiten, wie Entzündungsprozesse, proktologische Erkrankungen, Parodontose, chronische Entzündungserkrankungen der Genitalien oder Atrophie der Hörnerven eingesetzt werden.

Anhand von Zeichnungen werden Ausführungsbeispiele der Erfindung näher erläutert. Es zeigt:
- Fig. 1: die Einrichtung zur Behandlung von Erkrankungen der Sehbahn im Axialschnitt,
- Fig. 2: im Schnitt einen Teil der Einrichtung mit einem elastischen Element in der Form einer Spiralblattfeder,
- Fig. 3: den Schnitt III-III von Fig. 2,
- Fig. 4: im Schnitt einen Teil der Einrichtung mit einem elastischen Element in Form einer einfachen Blattfeder,
- Fig. 5: den Schnitt V-V von Fig. 4,
- Fig. 6: den Schnitt VI-VI von Fig. 1,
- Fig. 7: die Einrichtung von Fig. 1 in der Seitenansicht,
- Fig. 8: im Axialschnitt eine zweite Ausführungsform der Einrichtung,
- Fig. 9: den Schnitt IX-IX von Fig. 8, und
- Fig. 10 bis 12: die Bereiche am Kopf eines Menschen, die von einem Drehmagnet beeinflußbar sind.

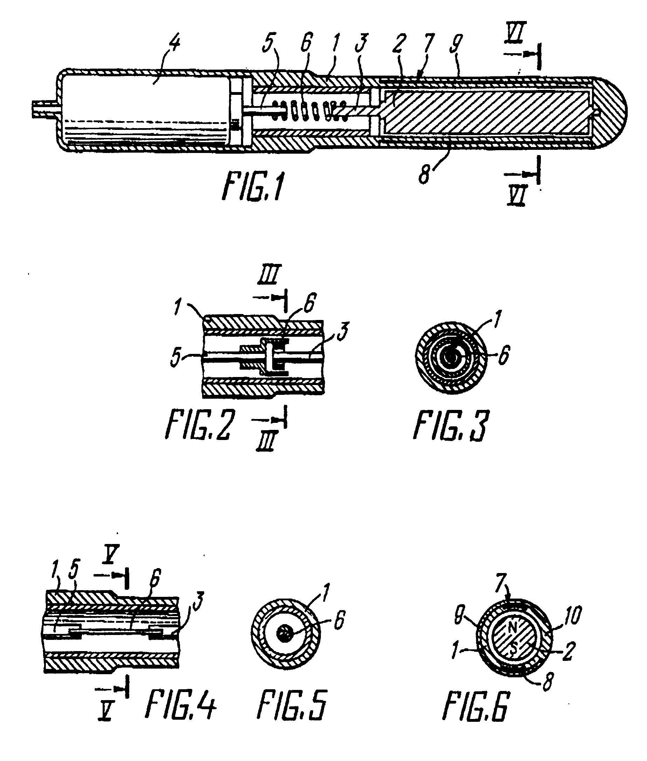

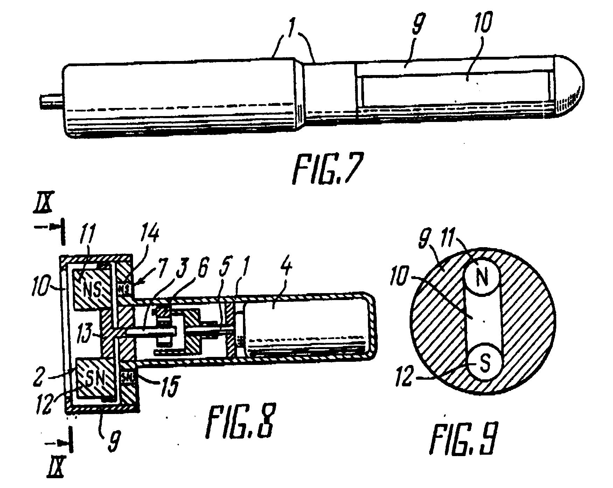

Die Einrichtung zur Behandlung der Erkrankungen der Sehbahn hat ein Gehäuse 1 (Fig. 1), in dem ein Hauptdauermagnet 2 mit einer Achse 3 und ein Antrieb 4, dessen Welle 5 mit der Achse 3 durch ein elastisches Element 6 verbunden ist, untergebracht sind. Das elastische element 6 kann in der Form einer zylindrischen Feder, einer Spiralblattfeder (Fig. 2, 3) oder einer einfachen Blattfeder (Fig. 4, 5) ausgeführt sein. Im Gehäuse 1 (Fig. 1) ist in der Wirkungszone des Magnetfeldes eine Magnetbremse 7 angeordnet, die in der Form zwei magnetischen Stahlstäbchen 8 (Fig. 6) ausgeführt ist.

Die Einrichtung zur Behandlung der Erkrankungen der Sehbahn hat eine Schirm 9 (Fig. 7), der ein Fenster 10 aufweist, der um Hauptdauermagnet 2 (Fig. 1) herum in der Wirkungszone des Magnetfeldes angebracht ist und aus einem Material von hoher elektrischer Leitfähigkeit ausgeführt ist.

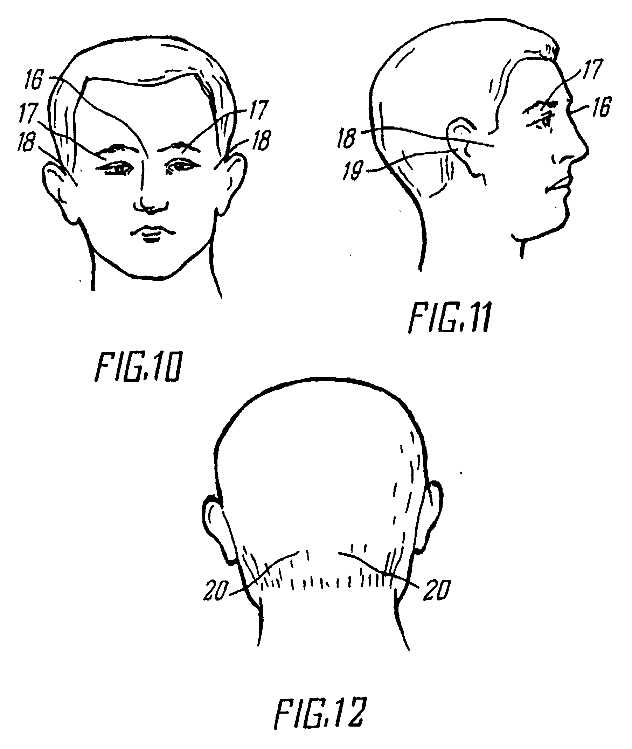

Betrachten wir eine Ausführungsvariante der Einrichtung zur Behandlung der Erkrankungen der Sehbahn, die in Fig. 8 dargestellt ist. In der Einrichtung ist der Hauptdauermagnet 2 aus zwei Magneten 11, 12 hergestellt, die mit ungleichnamigen Polen an einer Planscheibe 13, die beispielsweise aus einem ferromagnetischen Material ausgeführt ist, befestigt sind. Die Magnetbremse 7 ist aus zwei Dauermagneten 14, 15 hergestellt, die im Gehäuse 1 sich unter der Planscheibe 13 befinden, wobei die Magnetpole je umgekehrte Orientierung in bezug auf die Planscheibe 13 aufweisen. Das elastische Element 6 ist in der Form einer Spiralblattfeder ausgeführt. In Fig. 9 ist die Anordnung des Fensters 10 des Schirmes 9 gezeigt.

Die Einrichtung zur Behandlung der Erkrankungen der Sehbahn (Fig. 1) arbeitet wie folgt.

Die magnetischen Stahlstäbchen 8 der Magnetbremse 7 ermöglichen eine feste Orientierung der Pole des Hauptdauermagnetes 2 in bezug auf das Gehäuse 1. Mit dem Beginn der Drehung der Welle 5 des Antribs 4 fängt eine Verdrehung (ein Aufzeiehen) des elastischen Elementes 6 - der Feder - an, da die magnetischen Stahlstäbchen 8 der Magnetbremse 7 den Hauptdauermagnet 2 in unbeweglichem ("geknechtetem") Zustand halten.

Das elastische Element 6 - die Feder - wird aufgezogen, bis das Drehmoment an der Achse 3 des Hauptdauermagnetes 2 dem Moment gleich ist, das durch die Kupplung des Hauptdauermagnetes 2 mit den magnetischen Stahlstäbchen 8 gesichert ist. Nachdem das Drehmoment der aufgezogenen Feder - des elastischen Elementes 6 - und das Moment der Kupplung des Hauptdauermagnetes 2 mit den magnetischen Stahlstäbchen 8 gleich werden (aber die Welle 5 des Antriebs 4 rotiert noch), erfolgt ein eigenartiges "Abreißen" der Magnetbremse 7, daß heißt, der Hauptdauermagnet 2 beginnt sich zu drehen.

In dem Augenblick, wo der Ausgleich der Kräfte an der Achse 3 des Hauptdauermagnetes 2 von der Seite der aufgezogenen Feder und der Kupplung des Hauptdauermagnetes 2 mit den magnetischen Stahlstäbchen 8 der Magnetbremse 7 eintritt, ist die Winkelgeschwindichkeit bei der Drehung des Hauptdauermagnetes 2 gleich Null. Wenn das Rotieren der Welle 5 des Antriebs 4 sich fortsetzt und die Feder aufgezogen ist, beginnt der Hauptdauermagnet 2 sich zu drehen. Mit der Vergrößerung des Drehwinkels des Hauptdauermagnetes 2 vermindert sich dessen Kupplungskraft mit den magnetischen Stahlstäbchen 8 infolge einer Vergrößerung des Abstandes zwischen ihnen. Dabei beginnt die Winkelgeschwindigkeit bei der Drehung des Hauptdauermagnetes 2 zu wachsen. Bei der Umdrehung der Achse 3 des Hauptdauermagnetes 2 um 90° erweisen sich die Adhäsionskräfte der Gegenpole des Hauptdauermagnetes 2 mit den magnetischen Stahlstäbchen 8 als gleich, aber umgekehrt gerichtet, und die Gesamtadhäsionskraft ist gleich Null. Nach der Umdrehung der Achse 3 des Hauptdauermagnetes 2 um einen Winkel von über 90° wird die Adhäsionskraft dessen Pole mit den magnetischen Stahlstäbchen 8 zum Analogen eines Kräftepaares, weil sie mit der Drehrichtung zusammenfällt.

Nach der Ausgleichung der Winkelgeschwindigkeiten der Welle 5 des Antriebs 4 und der Achse 3 des Hauptdauermagnetes 2 fallen die Anziehungskräfte der Pole des Hauptdauermagnetes 2 und der magnetischen Stahlstäbchen 8 sowie die Kraft der sich losdrehenden Feder zusammen, und die Winkelgeschwindigkeit der Achse 3 des Hauptdauermagnetes 2 beginnt rasch anzuwachsen. In dem Augenblick des Durchgangs eines Umdrehungswinkels gleich 180° wird die Adhäsionskraft der Pole des Hauptdauermagnetes 2 maximal, die Umlaufbeschleunigung des Magnetfeldes wird auch maximal. Auch maximal wird dabei die Winkelgeschwindigkeit bei der Drehung der Achse 3 des Hauptdauermagnets 2 und entsprechend des Magnetfeldes. Dabei wird die Winkelgeschwindigkeit der Welle 5 des Antriebs 4 weniger als die Winkelgeschwindigkeit der Achse 3 des Hauptdauermagnets 2, und der Aufzugsgrad der Feder wird vermindert.

Nach der Umdrehung der Achse 3 des Hauptdauermagnetes 2 um einen Winkel von über 180° beginnt dessen Winkelgeschwindigkeit sich zu vermindern. Es wird dadurch erklärt, daß die Adhäsionskraft der Pole des Hauptdauermagnetes 2 mit den magnetischen Stahlstäbchen 8 maximal ist, die Feder hat sich losgedreht, die Winkelgeschwindigkeit der Welle 5 des Antriebs 4 ist geringer als die Winkelgeschwindigkeit der Achse 3 des Hauptdauermagnetes 2, und als bestimmende Drehkraft des Hauptdauermagnetes 2 erweist sich die von ihm gespeicherte kinetische Rotationenergie. Wenn die Wechselwirkung der Pole des Hauptdauermagnetes 2 dessen Rotationsträgheit gedämft hat, so fällt die Rotationsgeschwindigkeit bis auf Null ab , und die Adhäsionskraft des Hauptdauermagnetes 2 wird ihn zwingen, dessen Drehsinn durch einen umgekehrten zu wechseln. Es werden einige Drehschwingungen des Hauptdauermagnetes 2 um den dem Winkel 180° entsprechenden Punkt erfolgen. Diese Schwingungen werden dauern, bis die einseitig gerichtete Drehung der Welle 5 des Antriebs 4 wiederum die Feder bis zum Ausgleich durch die Adhäsionskräfte der Pole des Hauptdauermagnetes 2 mit den magnetischen Stahlstäbchen 8 aufzieht. Danach wird sich der Prozeß der Drehung des Hauptdauermagnetes 2 wiederholen.

In der Ausführungsvariante der Einrichtung, die in Fig. 8 angeführt ist, sind Dauermagneten 14 und 15 als Magnetbremse 7 benutzt. Diese Magneten sind diametral in bezug auf die Achse 3 der Drehung des Hauptdauermagnetes 2 angeordnet und in bezug auf die Planscheibe 13 mit den Gegenpolen orientiert. Die Planscheibe ist beispielsweise aus ferromagnetischem Material hergestellt.

Da zwischen den Dauermagneten 14 und 15 und der Planscheibe 13 eine Adhäsion besteht, die der obenbeschriebenen Variante analog ist, ist der Charakter der Formung der Drehung des Hauptdauermagnetes 2 (11, 12) an der Planscheibe 13 auch analog der obenbeschriebenen Variante.

Der Charakter der Drehung ändert sich einigermaßen, wenn die Planscheibe 13 aus einem nichtferromagnetischen Material ausgeführt ist.

In diesem Fall sichert die Variante der Nutzung der Magneten 14, 15, die mit gleichen Polen in bezug auf die Planscheibe 13 orientiert sind, keinen erwarteten Effekt der Zunahme der Rotationsgeschwindigkeit des Hauptdauermagnetes 2, wodurch die gestellte Aufgabe nicht in vollem Maße gelöst wird. Das wird dadurch erklärt, daß bei jeder beliebigen Größe des Drehwinkels bei der Wechselwirkung des Hauptdauermagnetes 2 (11, 12) und der Dauermagneten 14, 15 entgegengestzt gerichtete und gleich große Kräfte entstehen. Dabei wird die Lage des Hauptdauermagnetes 2 bezüglich des Gehäuses 1 nicht fixiert.

In dem Falle, wenn die Dauermagneten 14, 15 in bezug auf die Planscheibe 13 mit Gegenpolen orientiert sind, gibt es eine scharf ausgeprägte Fixierung der Lage des Hauptdauermagnetes 2 bezüglich des Gehäuses 1. Aber nach der Umdrehung um 90° tritt der Hauptdauermagnet 2 (11, 12) mit den Dauermagneten 14, 15 unter den Bedingungen der Abstoßung, das heißt der Bremsung der Drehung des Hauptdauermagnetes 2 (11, 12), in Wechselwirkung.

Das führt dazu, daß bei dem Drehwinkel 180° die Rotationsgeschwindigkeit des Hauptdauermagnetes 2 (11, 12) durch das Minimum durchgeht. Danach vollzieht sich der Prozeß des Anwachsens analog zur Variante der Einrichtung, die in Fig. 1. gezeigt ist. Die Roationsgeschwindigkeit in der vorliegenden Variante geht also im Prozeß der Umdrehung des Hauptdauermagnetes 2 (11, 12) um den Winkel 180° durch das Maximum bei 90° und das Minimum bei 180° durch und beginnt dann zu wachsen wie im Falle der Einrichtung, die in Fig. 1 gezeigt ist.

In der Variante der Ausführung der Einrichtung mit der Anwendung nur eines Dauermagnetes 14 oder 15, der mit einem beliebigen Pol in bezug auf die Planscheibe 13 orientiert ist, wird eine strenge Fixierung des Hauptdauermagnetes 2 (11, 12) bezüglich des Gehäuses 1 gesichert, derselbe Mechanismus und Charakter dessen Drehung wie im Falle einer ferromagnetischen Planscheibe 13.

Die wechselnde Rotationsgeschwindigkeit des Hauptdauermagnetes 2 (11, 12) kann bei großen Beschleunigungen in allen Fällen gesichert werden, ausschließlich der Variante mit der gleichen Orientierung der Pole der Dauermagnete 14, 15 bezüglich der Planscheibe 13, wenn sie aus nichtferromagnetischem Material ausgeführt ist.

Die Einrichtung zur Behandlung der Erkrankungen der Sehbahn wird in jeder Einwirkungsgegend 16 - 20 (Fig. 10 - 12) so eingestellt, daß die Rotierung des Hauptdauermagnetes 2 (11, 12) über dieser mit einer maximalen Änderung der Rotationsgeschwindigkeit gesichert wird.

Zwecks Verringerung der Streuflüsse des Magnetfeldes wird das Fenster 10 (Fig. 7, 9) des Schirmes 9 über der Einwirkungsgegend angeordnet.

Einwirkungsgegenden sind die Gegenden der Nasenwurzel 16 (Fig. 10, 11) und des oberen Teils 17 der Augenhöhle der beiden Augen bei eröffneten Lidern, die Schläfengegenden in Bereich des Außenrandes 18° der Augenhülle der beiden Augen, die beiden aurikulotemporalen Gegenden 19 auf dem Niveau der Proektion der Sehnervenkreuzung, die Gegend der Proektion der Sehanalysatoren auf den Inionen 20 (Fig. 12), wobei die Folge der Einwirkung auf diese Gegenden ist beliebig, die Einwirkungsdauer auf jede genannte Gegend beträgt 1 - 5 min individuell für jeden Patienten, maximale Induktion des Magnetfeldes beträgt 0,1 - 0,25 T auch individuell für jeden Patienten.

Die obenbeschriebenen Ausführungsvarianten der Einrichtung ermöglichen also eine Drehung des Magnetfeldes mit wechselnder Rotationsgeschwindigkeit und somit dessen Veränderung nach der Amplitude und Richtung, was eine Steigerung der Sehfunktionen und eine hoch wirksame Behandlung bei einer verkürzten Dauer ermöglicht.

Die erfindungsgemäße Einrichtung zur Behandlung der Erkrankungen der Sehbahn wird anhand folgender klinischer Beispiele näher erläutert.

### Beispiel 1

Eine Kranke nach der überstandenen Polyradikuloneuritis bemerkte eine Sehverschlechterung der beiden Augen. Diagnostiert ist eine retrobuläre Neuritis. Nach der konservativen Behandlung blieb das Sehvermögen des rechten Auges gleich 0,5 und das des linken Auges gleich 0,3 - 0,4. Die Gesichtsfelder beim Farbensehen sind um 20° enger. Nach den Angaben der automatisierten Perimetrie für OD - Verminderung der Lichtempfindlichkeit (bezogene Gesichtsfeldeinengung um 30° von dem Fixierpunkt: für OS - Einengung des Gesichtsfeldes auf der Nasenseite, eine Vielzahl von Relativskotomen in den Infranasenabschnitten des Sehfeldes). Elektrophysiologische Untersuchungen zeigten mäßige Änderungen in den inneren Schichten der Netzhaut und im Sehnerven.

Im Augenhintergrund: die Sehnervernscheibe ist blaß auf der temporalen Seite, die Arterien haben eine peitschenschnur - artige Schlängelung. Eine Makuladegeneration fehlt. Die Kranke vertrug 15 Prozeduren der Magnetstimulation von 1 min Dauer bei einer Induktion des Magnetfeldes von 0,1 T. Die Sehchärfe des rechten Auges steigerte sich auf 0,6, die des linken Auges - auf 0,6. Farbensehfelder erweiterten sich um 10°. Automatisiert Perimetrie nach sind die Relativskotome verschwunden, die Peripheriegrenzen erweiterten sich um 10°. Nach den Angaben einer elektrophysiologischen Untersuchung ist der Zustand der inneren Schichten der Netzhaut eine Norm, kleine Veränderungen im Sehnerven. Die Beobachtungdauer - 10 Monate.

### Beispiel 2

Die Kranke stellte eine Sehverschlechterung ab Juni 1986 fest. Diagnostiert ist eine rechtsseitige Makulodystrophie. Das Sehvermögen des rechten Auges 0,3 + 1,0 d - 0,5. Die "Schwelle" der elektrischen Sensibilität (100 µA) zeugt von mäßigen Veränderungen in den inneren Schichten der Netzthaut. Die Angaben zeugen von einer Funktionsstörung des Pigmentepithels und der Photorezeptoren vom exsudativen Charakter. Das Sehfeld ist konzentrisch um 30° eingeengt. Nach den Angaben der automatisierten Perimetrie ist eine Vielzahl von Parazentralskotomen, eine Einengung der Peripheriegrenzen um 30° nachgewisesn.

Die Kranke ist von einem Nervenarzt zur Erkennung der Ursache eine Netzhautdystrophie untersucht: vegetative Gefäßdystonie, belastet mit einer Osteochondrose; Angiospasmus von Gehirngefäßen mit einer Hochdruckkrankheit und Austausch-Polyarthritis in der Involutionsperiode.

Im Augenhintergrund: Sehnervenscheibe ist blaßrosa, die Grenzen sind deutlich, die Schlagadern sind verengert, peitschenschnurartig geschlängelt. An der Peripherie und in der Mittelpunktzone sind dystrophische Veränderungen in der Netzhaut (vom kystösen Typ) vorhanden. Die Kranke vertrug 15 Prozeduren der Magnetstimulation bei einer Prozedurdauer für jede Gegend von 2 min und einer Maximalinduktion des Magnetfeldes von 0,1 T. Die Kur wurde gut vertragen. In der Behandlungsperiode verschwunden die früher beunruhigten Kopfschmerzen. Die Sehschärfe des rechten Auges steigerte sich auf 0,88. Nach den Angaben der automatisierten Perimetrie kamen die Grenzen des Sehfeldes zu einer Norm zurück. Die elektrische Empfindlichkeitsschwelle verminderte sich auf 70 µA, was von unbedeutenden Veränderungen in inneren Schichten der Netzhaut zeugt.

### Beispiel 3

Der Kranke bemerkte, ein Jahr bevor er zum ersten Mal einen Arzt konsultiert hat, eine Sehverschlechterung beim rechten Auge. Das Sehvermögen wurde beeinträchtigt, weil der Blutkreislauf in den Gefäßen, die den Sehnerven speisen, gestört wurde. Nach der durchgeführten konservativen Therapie verbesserte sich das Sehvermögen nicht. Das Sehvermögen des rechten Auges 0,02 (ex), das des linken - 0,2 + 1,0 d = 0,6. Das Sehfeld des linken Auges ist konzentrisch auf 10° eingeengt, für das rechte Auge - fehlt auf der Nasenseite, auf der Temporalseite ist um 30° eingeengt. Nach den Ergebnissen elektrophysiologischer Untersuchungen - große Veränderungen in den inneren Schichten der Netzhaut und im Sehnerven.

Lauf der Computertomographie - der Durchmesser des rechten und linken Sehnerven 4,3 mm, die Dichte des rechten Nerven 21,3 H-Einheiten, die des linken - 47 H-Einheiten.

Augenhintergrund OD - Sehnervenscheibe: blaß, Grenzen: deutlich, Schlagadern: verengert, schlängeln sich peitschschnurartig.

Augenhintergrund OS - die Sehnervenscheibe ist weiß, die Grenzen sind deutlich, leichtes parapapillares Ödem. Die Arterien sind verengert, schlängeln sich peitschenschnurartig; die Venen sind mäßig erweitert.

An dem Kranken wurden 15 Behandlungseancen der Magnetstimulation während 5 min je Gegend durchgeführt, die Induktion des Magnetfeldes 0,1 T. Nach der Behandlung steigerte sich das Sehvermögen des rechten Auges auf 0,3 + 2,0 d = 0,88 das des linken auf 0,4 + 0,2 d = = 1,0.

Die Gesichtsfeldgrenzen wurden um 25° erweitert. Nach der Angabe elektrophysiologischer Untersuchungen - mäßige Veränderungen in inneren Schichten der Netzhaut und im Sehnerven. Die Beobachtungsdauer 1 Jahr. Die Sehschärfe bleibt wie früher, die Grenzen des Gesichtsfeldes haben sich um 5° eingeengt.

### Beispiel 4

Der Kranke bemerkte 6 Monate davor eine starke Sehverschlechterung beim linken Auge - diagnostiert ist eine ischämiebedingte Papillenschwellung. Konservative Behandlung machte keinen positiven Effekt.

Das Sehvermögen des linken Auges 0,01, korrigiert nicht, das des rechten gleich 0,3 + 2,5 d = 0,4.

Das Sehfeld des linken Auges ist um 35° konzentrisch eingeengt, das des rechten Auges - unveränderlich.

Nach der Angabe elektrophysiologischer Untersuchungen werden beträchtliche Veränderungen in den inneren Schichten der Netzhaut und im Sehnerven des linken Auges, mäßige Veränderungen in den inneren Schichten der Netzhaut und im Sehnerven des rechten Auges festgestellt.

Im Augenhintergrund: OS - die Sehnervenscheibe ist ödematös, die Grenzen zerfließen sich (sind nicht zu bestimmen), polymorphe Hämorrhagien in der Bewegungsrichtung des unteren Gefäßbündels und der oberen Makulaschlagader, die Makulagegend unverändert. Im Hintergrund des rechten Auges - die Sehnervenscheibe blaßrosa, die Grenzen sind deutlich, in der Makulagegend einzelne dystrophische Herde.

Dem Kranken wurde 15 Seancen der Magnetstimulation durchgeführt, die Seanoedauer für jede Gegend 1 min und die Induktion des Magnetfeldes 0,2 T. Nach der Behandlung stieg die Sehschärfe des linken Auges auf 0,1 + 3,0 d = 0,6 an, die Sehschärfe des rechten Auges steigerte sich auf 0,6. Das Gesichtsfeld des linken Auges erweiterte sich um 20°.

Nach der Angabe elektrophysiologischer Untersuchungen verbesserte sich der Zustand der inneren Schichten der Netzhaut und des Sehnerven, die Schwelle elektrischer Empfindlichkeit rechts - 40, links - 90, elektrische Labilität rechts - 40, links - 38.

Im Augenhintergrund links ist die Anzahl von Blutungen vermindert, frische Blutungen sind nicht nachgewiesen.

Die Dauer der Beobachtungen des Kranken 6 Monate, das Ergebnis stabil.

### Beispiel 5

Der Kranke bemerkte eine starke Sehverschlechterung des rechten Auges 1,5 Jahre vorher, das Sehen des linken Auges verschlechterte sich allmählich während 1,5 Jahre. In der Anamnese des Kranken eine Zuckerkrankheit.

Beim Konsultieren eines Okulisten ist eine Durchblutungsstörung in den den Sehnerven speisenden Gefäßen rechts und eine Angiosklerose und Makuladystrophie links diagnostiert.

Das Sehen des rechten Auges 0,01, korrigiert nicht, das des linken - 0,1 + 1,5 cyl - 1,5 = 0,2. Das Gesichtfeld rechts fehlt auf der Temporalseite, im Gesichtsfeld des linken Auges ist ein relatives Zentralskotom diagnostiert, konzentrische Einengung der Grenzen auf der Nasenseite auf 10°. Das Farbensehfeld ist um 20° links eingeengt, und rechts ist es um 20° für rote und blaue Farbe eingeengt, für grüne Farbe fehlt es.

Nach der Angabe elektrophysiologischer Untersuchungen sich die Schwelle elektrischer Empfindlichkeit und elektrische Labilität rechts nicht zu bestimmen, links sind mäßige Veränderungen in der Netzhaut und im Sehnerven nachgewiesen. Nach der Angabe einer Computertomographie sind atrophische Veränderungen der Sehnerven auf den beiden Seiten nachgewiesen. Eine Angiosklerose mit Kalzinose in der Region der inneren Kopfschlagader und der Augenschlagader. Ein Altershydrozephalus. Die Atrophie des Gehirnes. Nach der Angabe einer Ultraschall-Doppelerographie wird eine Störung der Durchblutung des Gehirnes und des Auges, stenosierende Prozesse infolge der Atherosklerose erkannt.

Dem Kranken wurde 10 Seancen der Magnetstimulation bei einer Dauer der Seance in jeder Gegend von 2 min durchgeführt, die Induktion des Magnetfeldes betrug 0,2 T. Nach der Behandlung steigerte sich die Sehschärfe des rechten Auges auf 0,3, die des linken - auf 1,0. Die Gesichtsfelder auf den beiden Seiten erweiterten sich um 20°, aus dem Gesichtsfeld des rechten Auges sind relative Skotome verschwunden. Die Grenzen des Gesichtsfeldes für die Farben haben sich um 10° erweitert.

Die Dauer der Beobachtung des Kranken 1 Jahr - die Ergebnisse der Behandlung sind stabil.

### Beispiel 6

Der Kranke hat ab 1982 J. ein Trauma des rechten Auges. Das Sehvermögen verminderte sich auf 0,4. Der Kranke ging eine Kur konservativer Behandlung durch - von der Behandlung wurde kein positiver Effekt erzielt.

Die Peripheriegrenzen des Gesichtsfeldes sind nicht verändert.Die Gesichtsfelder für rote und grüne Farbe sind auf 10° eingeengt, keine Reagierung auf die blaue Farbe. Nach der Angabe der Computerperimetrie wird eine geringe Herabsetzung der fovealen Lichtempfindlichkeit festgestellt - 34 db. Ein fast voller Ausfall der unteren Hälfte des Gesichtsfeldes bei relativer Einbehaltung der Mittelpunktzone. Nach der Angabe elektrophysiologischer Untersuchungen - mäßige Veränderungen der Funktionen der äußeren Schichten der Netzhaut, die Elektroretinographie ist vergrößert, ein indirektes Merkmal der Pathologie des Sehnerven.

Nach der Angabe der Ultraschall-Dopplerographie ist die Durchblutung rechts auf 1 ml/s gesenkt. Nach der Angabe der Computertomographie wird eine ungleichmäßige Verdünnung des rechten Sehnerven, an einigen Stellen auf 2,0 mm, festgestellt, seine Dichte ist auf 20 H-Einheiten herabgesetzt. Mäßige Verhärtungen der inneren Kopfschlagadern auf dem Niveau des Rückens des Türkensattels, der linke Sehnerv hat eine gewöhnliche Dicke und Dichte.

Dem Kranken wurde 15 Seancen der Magnetstimulation bei der Dauer der Seance von 5 min und einer Induktion des Magnetfeldes von 0,2 T durchgeführt. Das Sehvermögen wurde auf 0,7 wiederhergestellt.

Nach der Angabe der Computerperimetrie bleibt die foveale Empfindlichkeit auf 32 db herabgesetzt. Der Ausfall der unteren Grenze des Gesichtsfeldes auf 20°. Nach der Angabe der Dopplerographie wird eine Verbesserung der Durchblutung des rechten Auges erfaßt. Auf der Elektroretinographie bleiben mäßige Veränderungen in dem Mitelpunktbereich der Netzhaut.

Die Beobachtungsdauer 6 Monate - die Dynamik ist stabil.

### Beispiel 7

Dem Kranken ist eine Amblyopie des rechten Auges diagnostiert. Das Auge sieht schlecht von dem Alter 2 Jahre an.

Die Sehschärfe des rechten Auges 0,3, des linken-1,0. Nach der Angabe eines Dioptrimeters bezüglich OD - Hypermetropie + 1,0. Veränderungen im Gesichtsfeld und bei elektropgysiologischer Untersuchung sind nicht nachgewiesen.

Nach der Angabe der Computerperimetrie wurde eine Senkung der Lichtempfindlichkeit auf 28 db, eine Vergrößerung des blinden Fleckes und das Vorhandensein von relativen und absoluten Skotomen, meist auf der Nasenseite, erfaßt.

Dem Kranken wurde 15 Seancen der Magnetstimulation bei einer Dauer der Seancen in allen Gegenden je 1 min durchgeführt, die Induktion des Magnetfeldes beträgt 0,25 T. Nach der durchgeführten Behandlung stieg die Sehschärfe des rechten Auges auf 1,0. Die Angaben der Computerperimetrie und elektrophysiologischer Untersuchungen veränderten sich nicht.

Die Beobachtungsdauer - 10 Monate. Die Sehschärfe blieb hoch.

### Beispiel 8

Bei dem Kranken sieht das rechte Auge schlecht von der Kindheit an.

Ist zur Konsultation mit der Diagnose eingewiesen: Einwärtsschielen, Hypermetropie schwachen Grades, Ambliopie.

Das Sehvermögen des rechten Auges 0,03 + 1,5 d = = 0,05 - 0,1, das Sehvermögen des linken Auges 1,0. Der Augenhintergrund OD - in der Makulagegend - ausgeprägter Abbau des Pigmentepithels, über der fovealaren Gegend drei Herdchen von weißer Farbe mit deutlichen Grenzen.

Nach der Angabe elektrophysiologischer Untersuchungen - bedeutende Störungen der Funktion der äußeren Schichten der Netzhaut. Die Gesichtsfelder ohne Pathologie.

Dem Kranken wurden 15 Seancen der Magnetstimulation bei einer Dauer der Seancen in jeder Gegend von 2 min und einer Induktion des Magnetfeldes von 0,25 T durchgeführt. Im Ergebnis der durchgeführten Behandlung steigerte sich die Sehschärfe des rechten Auges auf 0,4. Daten elektrophysiologischer Untersuchungen und die Sehfelder sind ohne Dynamik.

Die Dauer der Beobachtung des Kranken 6 Monate. Die hohe Sehschärfe wird beibehalten.

### Beispiel 9

Die Kranke konsultierte den Arzt mit der Diagnose: teilweise Athropie des Sehnerven der beiden Augen nach der ein Jahr vorher vertragenen Arachnoiditis.

Die Sehschärfe des rechten Auges 0,6, des linken - 0,5, korrigiert nicht.

Nach der Angabe elektrophysiologischer Untersuchungen - grobe Veränderungen in den inneren Schichten der Netzhaut und im Sehnerven. Durchgeführt ist eine Ultraschalluntersuchung, Dopplerographie - beträchtliche Senkung der Hämodynmik des Sehnerven der beiden Augen. Die Gesichtsfelder für Farben sind auf 5° auf den beiden Seiten eingeengt, die Peripheriegrenzen des Gesichtsfeldes sind rechts auf 20°, links auf 40° eingeengt.

Nach der Angabe der Computertomographie wird eine Verminderung des Durchmessers der beiden Sehnerven auf 2,8 mm ohne wesentliche Veränderung der Dichte festgestellt. In der Proektion der rechten Kopfschlagader ist eine Verhärtung des Gewebes nachgewiesen. Die Zisternen der Hirnbasis sind infolge des mäßigen atrophischen Prozesses des Gehirnes erweitert. Der Kranken wurde 15 Seancen der Magnetstimulation bei einer Dauer der Seancen in jeder Gegend von 5 min und einer Induktion des Magnetfeldes von 0,25 T durchgeführt.

Die Sehschärfe steigerte sich auf 0,7. Die Grenzen des Peripheriesehfeldes erweiterten sich um 20°, das Farbensehen - um 10°. Nach der Angabe der Ultraschall-Dopplerographie wird eine Verbesserung der Durchblutung des Sehnerven festgestellt, obwohl sie noch niedrig ist.

Bei einer dynamischen Beobachtung bleibt die Sehschärfe während 7 Monate hoch.

### Beispiel 10

Der Kranken wurde die Diagnose: teilweise Atrophie der Sehnerven der beiden Augen festgestellt. Ein halbes Jahr vorher bemerkte sie eine starke Verschlechterung des Sehvermögens rechts nach der vertragenen hypertonischen Krise.

Die Sehschärfe des rechten Auges 0,2, des linken 0,9.

Im Augenhintergrund ist eine Papillenabblassung des Sehnerven auf den beiden Seiten, eine Angiosklerose festgestellt.

Nach der Angabe der Computerperimetrie ist eine Einengung des Gesichtsfeldes in den oberen Abschnitten, relative Skotome in dem nasalen Bereich der Gesichtsfelder - Merkmale einer Disfunktion der Sehnerven der Zentralgenese. Nach der Angabe der Computertomographie gibt es Verhärtungen des Gewebes, rechts mehr ausgeprägt, wo Petrifikate vorhanden sind.

Der Kranken wurde 10 Seancen der Magnetstimulation mit einer Dauer von je 4 min und einer Induktion von 0,1 T durchgeführt. Die Sehschärfe des rechten Auges steigerte sich auf 0,4, des linken auf 1,0. Nach der Angabe der Computerperimetrie ist eine beträchtliche positive Dynamik festgestellt. Die Ergebnisse elektrophysiologischer Untersuchungen - eine geringe Herabsetzung der elektrischen Labilität rechts (auf 38 ).

### Beispiel 11

Bei der Kranken ist die Diagnose festgestellt: eine lokale Atrophie des Sehnerven links nach der vertragenen Arachnoiditis. Die Sehschärfe des rechten Auges 1,0, des linken 0,7-0,8 . Im Augenhintergrund ist eine Abblassung der Sehnervenscheibe auf der Temporalseite der beiden Augen nachgewiesen.

Nach der Angabe elektrophysiologischer Untersuchungen - mäßige Veränderungen in den inneren Schichten der Netzhaut und im Sehnerven. Nach der Angabe der Computerperimetrie sind einzelne Skotome an der Peripherie des Gesichtsfeldes des rechten Auges und Skotome in der nasalen Hälfte des Feldes und auf dem vertikalen Meridian links nachgewiesen. Auf dem Computertomographen ist eine mäßige Hydrozephalie, eine multiple Sklerose im Stadium einer Remission, eine lokale Atrophie der Sehnerven beiderseitig bestimmt.

Der Kranken wurde 15 Seancen der Magnetstimulation von je 3 min Dauer mit einer maximalen Induktion von 0,1 T durchgeführt. Die Sehschärfe der beiden Augen 1,0. Die Computerperimetrie - die Lichtempfindlichkeit des gelben Fleckes stieg auf eine Norm an, es fehlt ein relatives Skotom auf der nasalen Seite links, aber ein absolutes Skotom auf dem vertikalen Meridian ist beibehalten. Ergebnisse elektrophysiologischer Untersuchungen - eine Norm.

### Beispiel 12

Einer Kranken ist die Diagnose festgestellt: eine lokale Atrophie der Sehnerven nach der von 10 Jahren überstandenen Arachnoiditis (die Kranke ist jetzt 18 Jahre alt).

Die Sehschärfe des rechten Auges 0,08, des linken 0,1 korrigiert nicht.

Der Augenhintergrund der beiden Augen: die Papille des Sehnerven ist blaß, die Grenzen sind ausgeprägt, die Streckung der Gefäße ist richtig.

Farbensehfelder: die rote Farbe - eingeengt, die blaue und grüne unterscheidet nicht. Nach der Angabe einer Computerperimetrie werden auf den beiden Augen vielzählige Parazentralskotome bestimmt, insbesondere im Bereich der Proektion des papillomakulären Bündals.

Der Kranken wurden 10 Seancen einer Magnetstimulation bei einer maximalen Induktion des Magnetfeldes von 0,25 T innerhalb von 4 min durchgeführt. Nach der Angabe einer Computerperimetrie bleiben relative Skotome im Mittelpunkt und im Bereich der Proektion des papillmakulären Bündels parazentral.

Die Anwendung der Einrichtung zur Behandlung der Erkrankungen der Sehbahn hat folgende Gegenanzeigen:
1. Das Vorhandensein von geschwulstähnlichen Bildungen in einem den magnetischen Einwirkungen ausgesetzten Gebiet.
2. Abschichtungen der Netzhaut.
3. Eitrige Iridozyklitis, Endophthalmitis, Panophthalmie.
4. Metallische Fremdkörper.
5. Ein hoher Augeninnendruck.
6. Ausgeprägte Vaskularisation einer Hornhautnarbe.

Die Anwendung der Einrichtung zur Behandlung der Erkrankungen der Sehbahn ermöglicht es:
1. Sehfunktionen in Abhängigkeit von der Ätiologie der Erkrankung auf das 2-15fache nach der Sehschärfe zu vergrößern, das Farbensehen bei dessen anfänglichem Fehlen wiederherzustellen, die Fläche der Skotome zu reduzieren und den Sehwinkel auf das 3fache zu vergrößern.
2. Die Wirksamkeit der Behandlung mit den Spätresultaten bis 1 Jahr beim Fahlen positiver Ergebnisse bei anderen Behandlungsverfahren (Laserbehandlung, pharmakologische Heilung, vasorekonstruktive Operationen, Akupunktur und Elektropunktur) zu steigern.
3. Die Dauer der Behandlung bis 15 Tagen zu verkürzen.-1 Zyklus ist 15 Seancen der Magnetotheraphie.

## Patentansprüche

1. Einrichtung zur Behandlung von Erkrankungen, insbesondere der Sehbahn, mit einem Gehäuse (1), in dem ein Antrieb (4) und mindestens ein drehbarer Hauptdauermagnet (2) untergebracht sind, dessen Achse (3) mit einer Welle (5) des Antriebs (4) verbunden ist, **gekennzeichnet** durch eine Magnetbremse (12), die im Gehäuse (1) im Bereich der Einwirkung des Magnetfeldes des Hauptdauermagneten untergebracht ist, wobei die Achse (3) des Hauptdauermagneten (2) mit der Welle (5) des Antriebs (4) durch ein elastisches Element (11) verbunden ist.

2. Einrichtung nach Anspruch 1, dadurch **gekennzeichnet,** daß die Magnetbremse (12) in der Form von mindestens einem magnetischen Stahlstäbchen (13) ausgeführt ist.

3. Einrichtung nach Anspruch 1, dadurch **gekennzeichnet,** daß die Magnetbremse (12) in der Form von mindestens einem zusätzlichen Dauermagneten (19, 20) ausgeführt ist.

4. Einrichtung nach Anspruch 1, **gekennzeichnet** durch einen ein Fenster, (10) aufweisenden Schirm (14), der um den Hauptdauermagnet (2) herum angeordnet ist und aus einem Material mit einer hohen elektrischen Leitfähigkeit besteht.

## Claims

1. A device for the treatment of diseases, particularly of the ocular system, with a casing (1), in which a driving apparatus (4) and at least one rotatable main permanent magnet (2) are housed, the spindle (3) of the magnet being connected to a shaft (5) on the driving apparatus (4), **characterised by** a magnet brake (12) housed in the casing (1) in the region of effect of the magnetic field of the main permanent magnet, the spindle (3) of the main permanent magnet (2) being connected to the shaft (5) on the driving apparatus (4) by means of a resilient member (11).

2. A device according to claim 1, **characterised in that** the magnet brake (12) is in the form of at least one small magnetic steel rod (13).

3. A device in accordance with claim 1, **characterised in that** the magnet brake (12) is in the form of at least one additional permanent magnet (19, 20).

4. A device in accordance with claim 1, **characterised by** a screen (14) having an opening (10), and arranged surrounding the main permanent magnet (2), and consisting of a material with a high electric conductivity.

## Revendications

1. Dispositif pour le traitement de maladies, notamment du système oculaire, avec un boîtier (1) dans lequel sont logés un dispositif d'entraînement (4) et au moins un aimant permanent principal rotatif dont l'axe (3) est relié à l'arbre (5) du dispositif d'entraînement (4), caractérisé par un frein magnétique (12) qui est logé dans le boîtier (1) dans zone de l'action du champ magnétique de l'aimant permanent principal, l'axe (3) de l'aimant permanent principal (2) étant relié à l'arbre (5) du dispositif d'entraînement (4) par un élément élastique (11).

2. Dispositif selon la revendication 1, caractérisé en ce que le frein magnétique (12) est réalisé sous forme d'au moins un bâtonnet magnétique (13) en acier.

3. Dispositif selon la revendication 1, caractérisé en ce que le frein magnétique (12) est réalisé sous forme d'au moins un aimant permanent supplémentaire (19, 20).

4. Dispositif selon la revendication 1, caractérisé par un écran (14) pourvu d'une fenêtre (10) et qui est disposé autour de l'aimant permanent principal (2) et réalisé en un matériau ayant une conductibilité électrique élevée.
